Europäisches Patentamt

European Patent Office

Office européen des brevets

(11) Veröffentlichungsnummer: **0 354 172**
A1

## (12) EUROPÄISCHE PATENTANMELDUNG

(21) Anmeldenummer: **89810458.3**

(22) Anmeldetag: **14.06.89**

(51) Int. Cl.⁵: **B 65 B 9/04**
B 65 B 63/02, B 65 D 85/16

(30) Priorität: **26.07.88 CH 2842/88**

(43) Veröffentlichungstag der Anmeldung:
**07.02.90 Patentblatt 90/06**

(84) Benannte Vertragsstaaten:
**CH DE FR GB IT LI**

(71) Anmelder: **Leone, Demetrio**
**Veilchenweg 17**
**CH-8437 Zurzach (CH)**

(72) Erfinder: **Leone, Demetrio**
**Veilchenweg 17**
**CH-8437 Zurzach (CH)**

(74) Vertreter: **Breiter, Heinz**
**Patentanwalt H. Breiter AG Wartstrasse 4 Postfach 1163**
**CH-8401 Winterthur (CH)**

(54) **Verpackung von saugfähigen Gegenständen, insbesondere Windeln.**

(57) Aus einer verformbaren Folie (1) werden Behälter (9) mit nach aussen gebogenden Rändern (9') ausgeformt, bevorzugt tiefgezogen. Saugfähige Gegenstände, insbesondere Windeln, werden einzeln oder stapelweise in den Behältern (9) zusammengepresst, wobei das Innere dieser Pakete (4) vorzugsweise evakuiert wird. Das Zusammenpressen erfolgt durch eine Pressplatte (12), mit einem zwischenliegenden Deckband (11). Rahmen (15) verkleben oder versiegeln das Deckband (11) mit den Rändern (9') der Behälter (9).

Mehrere Pakete (4), die aus zwei verklebten oder versiegelten Kunststoffolien (1, 11) bestehen, bilden zweckmässig eine Trageinheit und die Anzahl der Pakete (4) und der saugfähigen Gegenstände in einem Paket (4) werden den praktischen Bedürfnissen angepasst. Es werden z.B. vier Pakete mit je zehn Stück Windeln an einer Folie angeordnet, die als eine Tragtasche ausgebildet sein kann.

FIG.3

EP 0 354 172 A1

**Beschreibung**

## Verpackung von saugfähigen Gegenständen, insbesondere Windeln

Die Erfindung bezieht sich auf ein Verfahren zur Verpackung von wenigstens im Teil ihres Volumens saugfähigen Gegenständen und auf eine Anwendung des Verfahrens.

Es sind verschiedene Arten von saugfähigen Gegenständen bekannt. Für die Körperpflege werden z.B. Babywindeln verwendet, es gibt jedoch auch Windeln für kranke Erwachsene. Diese Windeln haben vor allem saugfähige Papierschichten und/oder saugfähiges Pulver und eine untere undurchlässige Folie mit Befestigungsmitteln. Es sind auch verschiedene saugfähige Gegenstände für Frauenhygiene bekannt. Ebenfalls Papiertaschentücher und bestimmte Arten von Klosettpapier können den genannten saugfähigen Gegenständen zugerechnet werden.

Der gemeinsame Nachteil der bekannten saugfähigen Gegenstände besteht darin, das sie viel Raum beanspruchen, so dass die Lagerung und der Transport unbequem und unwirtschaftlich sind. Insbesondere für Reisen ist das grosse Volumen sehr hinderlich.

Der vorliegenden Erfindung liegt die Aufgabe zugrunde, die Nachteile des Bekannten zu beseitigen und ein Verfahren zur Verpackung dieser Gegenstände zu schaffen, das die Grösse der verpackten, saugfähigen Gegenstände wesentlich vermindern und somit den Transport und die Lagerung nicht nur beim Verkauf, sondern hauptsächlich bei dem Verbraucher erleichtert. Die Anwendung der Erfindung soll eine hygienische und das Volumen der Gegenstände vermindernde Verpackung von Einzelstücken und Stapeln ermöglichen.

In bezug auf das Verfahren wird die Aufgabe erfindungsgemäss dadurch gelöst, dass
- aus einer verformbaren Folie Behälter mit nach aussen gebogenen Rändern ausgeformt,
- in diese Behälter je ein Stapel der saugfähigen Gegenstände eingelegt,
- ein Deckband aus einer Folie über diese Stapel der saugfähigen Gegenstände geführt,
- die saugfähigen Gegenstände über das Deckband mit einer Pressplatte zusammengepresst werden, bis das Deckband die nach aussen gebogenen Ränder der Behälter berührt oder wenigstens oberhalb der Behälter verläuft,
- und danach geheizte Rahmen das Deckband mit den Rändern der Behälter in die Form von Paketen verkleben oder versiegeln.

Der Vorteil der Erfindung ist darin zu sehen, dass durch das Zusammenpressen und Evakuieren das Volumen der saugfähigen Gegenstände abhängig von der Art der Gegenstände etwa um 40 bis 50 % vermindert wird, wobei das Versiegeln in den zwei Folien eine sehr vorteilhafte und hygienische, gegen Feuchtigkeit und Staub schützende Verpackung bildet. Ein Stapel von saugfähigen Gegenständen kann durch einen einzelnen Gegenstand ersetzt sein. Einfachheitshalber wurde und wird nachfolgend nur von einem Stapel gesprochen, es sind jedoch anstelle eines Stapels immer Einzelstücke möglich.

Die Behälter werden vorzugsweise tiefgezogen und ggf. anschliessend abgestreckt. Die Deckfolie wird bevorzugt auf die Ränder der Behälter gesiegelt, unter Verwendung eines geheizten oder heizbaren Rahmens.

Es ist zweckmässig, die zu verklebenden oder zu versiegelnden Pakete vor und/oder während des Verschliessens zu evakuieren. Sowohl das Zusammenpressen als auch das Evakuieren weisen denselben Zweck auf, d.h. die sich im Paket befindende Luft soll entfernt werden und der Druck von aussen (entweder mechanischer oder atmosphärischer Druck) vermindert auch das Volumen der zusammenpressbaren Gegenstände, z.B. der Windeln.

Bevorzugt werden die Stapel der saugfähigen Gegenstände teilweise zusammengepresst bevor das Deckband darüber geführt wird. Das Leiten der Stapel wird im teilweise zusammengepressten Zustand erleichtert und die Höhe des Deckbands und der Pressplatten muss nicht so hoch sein.

Zweckmässig werden die nach dem versiegeln des Deckbands mit den Rändern des Behälters entstandenen Pakete mit einer Perforation des Deckbands und/oder der tiefziehbaren Folie versehen. Dies ermöglicht, aus einer grösseren Packung Einzelpakete mit einem oder mehreren Gegenständen herauszunehmen, ohne die Qualität der Verpackung der restlichen Pakete zu vermindern.

Die Pakete weisen bevorzugt eine Aufreissschnur oder eine längliche Aufreissöffnung auf. Diese Massnahme erleichtert ein schnelles und bequemes Herausnehmen eines Gegenstandes, z.B. einer Windel aus dem Paket.

Zweckmässig werden die tiefgezogenen Behälter aus den Formenvertiefungen mit Ausstossstiften entfernt. Dies ermöglicht eine schnelle Fertigung der Behälter, ohne diese zu beschädigen.

Nach einer besonderen Ausführungsform werden die aus der verformbaren Folie hergestellten Behälter voneinander getrennt, in einer vorgegebenen Verteilung auf eine Grundplatte geführt, nach dem Zusammenpressen der Stapel der Gegenstände werden diese Behälter mit dem verklebenden oder versiegelnden Deckband in gewählten Entfernungen gehalten. Diese Ausführungsform wird vorzugsweise bei einer verformbaren Folie angewendet, die dicker und teurer ist als das Deckband, nur für die Herstellung der Behälter. Als Verbindungsfolie der einzelnden Pakete dient nur das dünnere und billigere Deckband.

Zweckmässig wird das Deckband mit Oeffnungen für einen Handgriff versehen und in Form von Tragtaschen mit den Paketen geschnitten. Es ist selbstverständlich möglich, das tragende Deckband nur mit einer Oeffnung und Handgriff versehen und so z.B. vier, acht oder zwölf Pakete hängend zu tragen. In bezug auf die durch das Zusammenpressen gewonnene kleine Dicke der Pakete besteht jedoch die günstige Möglichkeit, die Oeffnungen für den Handgriff auf beiden Enden des Abschnittes herzustellen, das Band dann zusammenzuklappen so dass sich beide Oeffnungen überdecken und gemeinsam den Handgriff bilden. Somit wird in derselben Höhe eine doppelte Anzahl von verpackten Gegenständen getragen.

Das Verfahren wird erfindungsgemäss angewendet für eine Verpackungseinheit mit wenigstens einem Paket, welches die saugfähigen Gegenstände einzeln oder in Gruppen enthält.

Für den Hausgebrauch oder für eine Kinderklinik werden grössere Packungen von Windeln bevorzugt. Demgegenüber reicht für eine kürzere Reise eine kleinere Anzahl aus, so dass entweder direkt eine kleinere Einzelpackung verwendet wird oder dass aus einer grösseren Packung mit mehreren Einzelpaketen eines oder mehrere dieser Einzelpakete herausgenommen werden.

Eine günstige Variante ist darin zu sehen, dass in einer Verpackungseinheit mehrere Pakete mit verschiedener Anzahl von Gegenständen pro Paket enthalten sind. So werden z.B. für den Hausgebrauch Pakete mit grösserer Anzahl verwendet, für Reisen Pakete mit kleinerer Anzahl oder sogar Einstückpakete.

Die Erfindung wird anhand der in der Zeichnung dargestellten Ausführungsbeispiele näher erläutert. Es zeigen schematisch:
- Fig. 1 eine Draufsicht auf eine aufgeklappte Verpackung mit vier Paketen,
- Fig. 2 eine perspektivische Ansicht eines offenen Behälters,
- Fig. 3 eine Darstellung der Herstellung der Behälter, wobei eine verformbare Folie über die ganze Länge der Verpackungsbahn geführt wird,
- Fig. 4 die Fortsetzung der Fig. 3, in der das Abschneiden, Stanzen und Perforieren symbolisch angedeutet ist,
- Fig. 5 eine Tragtasche mit vier Paketen in teilweise zusammengeklapptem Zustand,
- Fig. 6 dieselbe Tragtasche aus Fig. 5 in voll zusammengeklapptem Zustand,
- Fig. 7a das Tiefziehen und Abschneiden eines einzelnen Behälters,
- Fig. 7b die weitere Bewegung der einzelnen Behälter inkl. dem Verschliessen mit dem durchgehenden Deckband, und
- Fig. 8 das Schneiden, Stanzen und Perforieren einer fertigen Einheit.

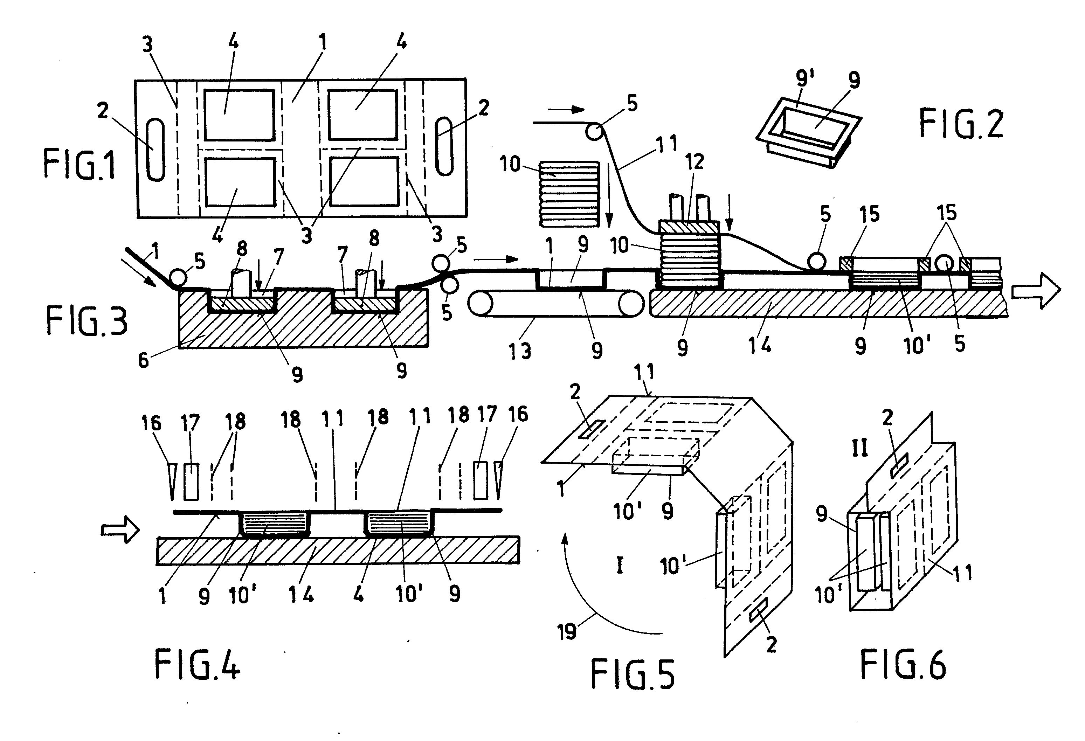

Fig. 1 zeigt einen Teil einer verformbaren Folie 1, die auch als Unterband bezeichnet wird. Die verformbare Folie 1 besteht z.B. aus Polyäthylen/Nylon 40/100. Seitlich sind zwei Oeffnungen 2 für einen Handgriff ausgestanzt und entlang der Pakete 4, die in diesem Beispiel mehrere Windeln enthalten, aber auch als Verpackung für ein Stück dienen können, ist die Perforation 3 geführt, die ein leichtes Herausnehmen eines Paketes 4 ermöglicht.

In Fig. 2 ist ein einzelner Behälter 9 dargestellt, der mit nach aussen gebogenen Rändern 9' versehen ist.

Die Herstellung der Behälter 9 ist im Schnitt in Fig. 3 gezeigt. Für das in Fig. 1 gezeigte Beispiel werden gleichzeitig vier Behälter ausgeformt, im vorliegenden Beispiel durch Tiefziehen. In Matrize 6 befinden sich vier Formenvertiefungen 7, von denen im Schnitt nur zwei sichtbar sind. Die verformbare Folie 1 wird über eine Umlenkrolle 5 zugeführt und mit den Stempeln 8 in die Formenvertiefungen 7 abgesenkt. Weil sowohl die Matrize 6 als auch die Stempel 8 geheizt sind, formt sich die Folie 1 entsprechend der Form und nach Entfernen der Stempel 8 wird sie mit den weiteren Rollen 5 aus den Formenvertiefungen 7 mit tiefgezogenen Behältern 9 herausgezogen. Die Behälter 9 bilden immer noch Bestandteile der tiefziehbaren Folie 1 und werden mit einem endlosen Band 13 weitertransportiert. Von oben wird ein Stapel 10 von Windeln zugeführt, zum Beispiel die Windeln einmal gefaltet. Nach einer andern Variante ist selbstverständlich auch ein dreifaches oder sogar vielfaches Falten ohne Beschädigung des Materials möglich. Von oben wird ein Deckband 11 über ein Rollensystem 5 über die obere Fläche des Stapels 10 geleitet. Das Deckband 11 besteht aus Polyäthylen/Nylon 20/70. Der Stapel 10 mit dem entsprechenden Teil des Deckbands 11 wird mit einer Pressplatte 12 in den Behälter 9 gepresst und teilweise oder ganz gefüllt. Die tiefziehbare Folie 1 (Unterband) wird weiter bewegt und dann ist sie auf einer festen Grundplatte 14 abgestützt. Der Stapel 10 ist zusammengepresst mit 10 bezeichnet.

Geheizte Rahmen 15 verschweissen das Deckband 11 mit den Rändern 9' des Behälters 9, wobei zweckmässig vor dem Zusammenschweissen und eventuell auch während des Zusammenschweissens das Innere des Behälters 9 evakuiert wird.

In Fig. 4, der Fortsetzung von Fig. 3, ist die Phase dargestellt, in der das Unterband 1 und das Deckband 11 mit den zusammengeschweissten, evakuierten Paketen 4 mit zusammengepressten Stapeln 10' mit Hilfe von Messern 16 getrennt werden. Stanzkörper 17 zeigen symbolisch Werkzeuge für Fertigung der Oeffnungen 2 für den Handgriff. Mit gestrichelten Strichen ist die Funktion der Perforiermesser 18 angedeutet.

Fig. 5 zeigt die erste Phase des Zusammenklappens der Tragtasche. Mit 19 ist die Richtung des Zusammenklappens gezeigt. Die in Fig. 6 gezeigte zweite Phase stellt eine fertige Tragtasche mit vier Windelpaketen dar.

Bei dem bisher beschriebenen Verfahren wurde über die ganze Länge des Bands sowohl das dickere Unterband als auch das dünnere Deckband 11 verwendet. Weil die mechanische Festigkeit des Deckbands ausserhalb des Behälters zum Bilden einer Tragtasche ausreichend ist, wird gemäss einer Weiterentwicklung der Erfindung das dickere Unterband nur für die Behälter 9 und für ihre nach aussen gebogenen Ränder 9' angesetzt. Das Verfahren entspricht im wesentlichen dem beschriebenen, es gibt jedoch kleine Unterschiede.

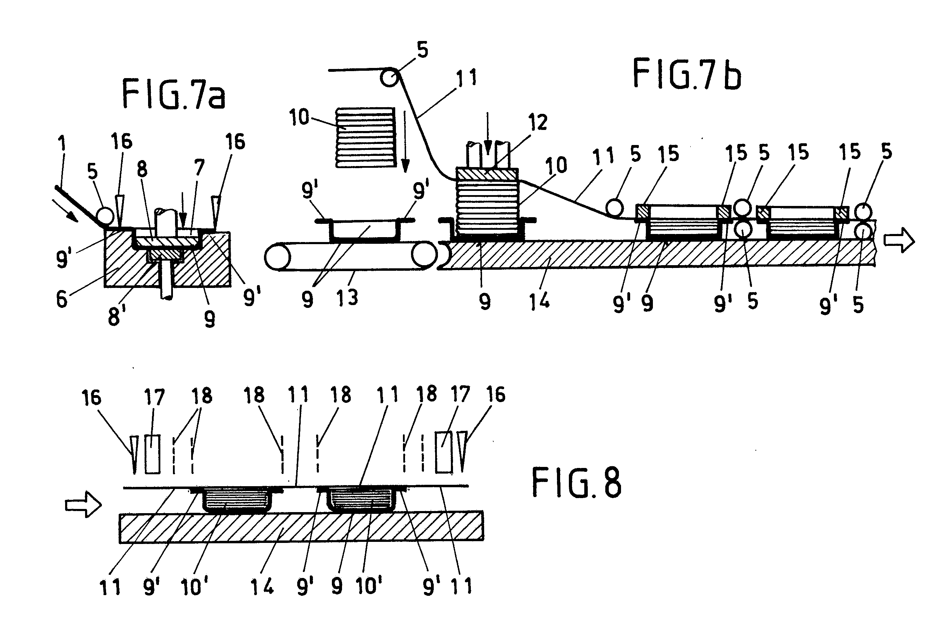

Gemäss Fig. 7a wird ein Behälter 9, in schon beschriebener Weise mit dem Stempel 8 tiefgezogen und gleichzeitig mit Messern 16 abgeschnitten, so dass der zum Verschweissen mit dem Deckband 11 bestimmte Rand 9' gebildet wird. Mit einem Ausstossstift 8' wird der Behälter 9 aus der Formenvertiefung 7 herausgestossen und weiter mit nicht dargestellten Rollen 5, die den Rand 9' des Behälters 9 halten bzw. transportieren, auf das endlose Band 13 geschoben. Die Verteilung der Behälter 9 auf dem endlosen Band 13 und auf der Grundplatte 14 ist so programmiert, dass deren Entfernung der Entfernung der Pakete 4 des fertigen Erzeugnisses entspricht. Wie schon anhand von Fig. 3 beschrieben, wird wenigstens ein Stapel 10 von Windeln in den oder in die Behälter 9 eingeführt, mit der Pressplatte 12 über das eingeführte Deckband 11 zusammengepresst und mit dem geheizten Rahmen 15 nach und/oder während der Evakuierung versiegelt.

Fig. 8 entspricht im wesentlichen Fig. 4, mit dem Unterschied, dass in der Fig. 8 nur das Deckband 11 durchgehend geführt ist und das dickere Unterband 1 dagegen nur die Be hälter 9 und ihre Ränder 9' bildet. Auch da werden Messer 16, Stanzkörper 17 und Perforiermesser 18 verwendet und am Ende wird gemäss schon beschriebenen Fig. 5 und 6 eine Tragtasche mit mehreren, in diesem Fall mit vier einzeln, vorzugsweise vakuumverpackten Paketen gefaltet, die zusammengepresste Stapel 10' von Windeln enthalten. In diesem Beispiel enthält ein Paket einen Stapel 10 mit zehn Windeln.

Als Beispiel wurde die Verpackung von Windeln dargestellt, es kommen jedoch auch andere saugfähige Gegenstände für die Hygiene in Frage. Die praktischen Prüfungen haben gezeigt, dass das Volumen der verwendeten Windeln durch das erfindungsgemässe Verfahren auf etwa 50% vermindert werden kann. Die Verminderung des Volumens ist für Transport und Lagerung sehr vorteilhaft und zwar sowohl bei der Herstellung als auch beim Handel und prakischer Benutzung.

**Patentansprüche**

1. Verfahren zur Verpackung von wenigstens im Teil ihres Volumens saugfähigen Gegenständen, insbesondere Windeln,
dadurch gekennzeichnet, dass
- aus einer verformbaren Folie (1) Behälter (9) mit nach aussen gebogenen Rändern (9') ausgeformt,
- in diese Behälter (9) je ein Stapel (10) der saugfähigen Gegenstände eingelegt,
- ein Deckband (11) aus einer Folie über diese Stapel (10) der saugfähigen Gegenstände geführt,
- die saugfähigen Gegenstände über das Deckband (11) mit einer Pressplatte (12) zusammengepresst werden, bis das Deckband (11) die nach aussen gebogenen Ränder (9') der Behälter (9) berührt oder wenigstens oberhalb der Behälter (9) verläuft, und
- danach Rahmen (15) das Deckband (11) mit den Rändern (9') der Behälter (9) in die Form von Paketen (4) verkleben oder versiegeln.

2. Verfahren nach Anspruch 1, dadurch gekennzeichnet, dass die Behälter (9) tiefgezogen, und die Pakete (4) vor und/oder während des Verschliessens vorzugsweise evakuiert werden.

3. Verfahren nach Anspruch 1 oder 2, dadurch gekennzeichnet, dass vor dem Führen des Deckbands (11) über die Stapel (10) der saugfähigen Gegenstände diese teilweise zusammengepresst werden.

4. Verfahren nach einem der Ansprüche 1 - 3, dadurch gekennzeichnet, dass die nach dem Verkleben oder Versiegeln des Deckbands (11) mit den Rändern (9') entstandenen Pakete (4) mit einer Perforation (3) des Deckbands (11) und/oder der verformbaren Folie (1) versehen werden.

5. Verfahren nach einem der Ansprüche 1 - 3, dadurch gekennzeichnet, dass die Pakete (4) mit je einer Aufreissschnur oder einer länglichen Aufreissöffnung versehen werden.

6. Verfahren nach einem der Ansprüche 2 - 5, dadurch gekennzeichnet, dass die tiefgezogenen Behälter (9) mit Ausstossstiften (8') aus den Formenvertiefungen (7) entfernt werden.

7. Verfahren nach einem der Ansprüche 1 - 6, dadurch gekennzeichnet, dass die aus der verformbaren Folie (1) hergestellten Behälter (9) voneinander getrennt in einer vorgegebenen Verteilung auf eine Grundplatte (14) geführt, und nach dem Zusammenpressen der Stapel (10) der Gegenstände diese Behälter (9) mit einem verklebenden oder versiegelnden Deckband (11) in gewählten Entfernungen gehalten werden.

8. Verfahren nach einem der Ansprüche 1 - 7, dadurch gekennzeichnet, dass das Deckband (11) mit Oeffnungen (2) für einen Handgriff versehen, mit den Paketen (4) geschnitten und in Form von Tragtaschen an den Enden verbunden wird.

9. Anwendung des Verfahrens nach einem der Ansprüche 1 - 8 zur Herstellung einer Verpackungseinheit, mit wenigstens einem Paket (4), welches die saugfähigen Gegenstände einzeln oder in Gruppen enthält.

10. Anwendung des Verfahrens nach Anspruch 9 für eine Verpackungseinheit, die mehrere Pakete (4) mit verschiedener Anzahl von Gegenständen pro Paket (4) enthält.

FIG.1

FIG.2

FIG.3

FIG.4

FIG.5

FIG.6

I

II

EP 0 354 172 A1

FIG.7a

FIG7b

FIG.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | FR-A-2 202 498 (ETS BOUTILLIER) <br> * Insgesamt * | 1,9 | B 65 B 9/04 <br> B 65 B 63/02 <br> B 65 D 85/16 |
| Y | | 2-4,7,8 | |
| | --- | | |
| Y | FR-A-2 036 965 (SOCIETE D'APPLICATION PLASTIQUE, MECANIQUE ET ELECTRONIQUE) <br> * Seite 4, Zeile 34 - Seite 5, Zeile 5; Figur 1 * | 2 | |
| | --- | | |
| Y | DE-A-3 323 061 (D.C. SPANN) <br> * Ansprüche 1,3 * | 3 | |
| A | | 1,2 | |
| | --- | | |
| A | EP-A-0 046 739 (A. STUMSNER) <br> * Anspruch 7; Figur 1 * | 1 | |
| | --- | | |
| A | EP-A-0 266 489 (AM INTERNATIONAL) <br> * Seite 7, letzter Absatz; Figur 7 * | 1 | |
| | --- | | |
| Y | US-A-3 605 374 (D.C. MUELLER et al.) <br> * Spalte 2, Zeilen 59-63; Spalte 3, Zeilen 35-74; Figuren 1,4-7 * | 4 | RECHERCHIERTE SACHGEBIETE (Int. Cl.5) |
| | --- | | B 65 B <br> B 65 D |
| Y | US-A-3 652 363 (W.G. KINSLOW) <br> * Figur 1 * | 7 | |
| | --- | | |
| Y | FR-A-1 511 072 (M.B. GILLAIN) <br> * Figuren 3-6 * | 8 | |
| | ----- | | |

Der vorliegende Recherchenbericht wurde für alle Patentansprüche erstellt

| Recherchenort | Abschlußdatum der Recherche | Prüfer |
|---|---|---|
| DEN HAAG | 26-10-1989 | SCHELLE,J. |